# EUROPEAN PATENT SPECIFICATION

(11) **EP 3 004 040 B1**
(45) Date of publication and mention of the grant of the patent: **20.06.2018**
(21) Application number: 14730105.5
(22) Date of filing: 28.05.2014
(51) Int. Cl.: C07C 29/86

(54) **PROCESS FOR THE SEPARATION OF 1,4-BUTANEDIOL**
VERFAHREN ZUR TRENNUNG VON 1,4-BUTANDIOL
PROCÉDÉ DE SÉPARATION DE 1,4-BUTANEDIOL

(30) Priority: 31.05.2013 EP 13169988
(43) Date of publication of application: 13.04.2016
(73) Proprietor: Shell Internationale Research Maatschappij B.V., 2596 HR The Hague (NL)
(72) Inventor: KOOT, Wouter, 1031 HW Amsterdam (NL); WADMAN, Sipke Hidde, 1031 HW Amsterdam (NL); LANGE, Jean Paul Andre Marie Joseph Gishlain, 1031 HW Amsterdam (NL); FISCHER, Kai Jürgen, 1031 HW Amsterdam (NL)
(74) Representative: Shell Legal Services IP
(86) International application number: PCT/EP2014/061182
(87) International publication number: WO 2014/191522

(56) References cited:
- US-A- 4 032 583
- US-A- 4 447 643

## Description

### Field of the Invention

The present invention relates to a process for the separation of 1,4-butanediol.

### Background of the Invention

1,4-butanediol (1,4-BDO) is a valuable chemical used industrially as a solvent and in the production of elastic fibres such as elastane/spandex, polybutyrate terephthalate and derivatives of gamma butyrolactone.

It is produced industrially via a number of routes from petrochemical feedstocks, obtainable from fossil fuels. One industrial route requires the reaction of acetylene with two equivalents of formaldehyde followed by hydrogenation of the resultant 1,4-butynediol to form 1,4-butanediol. In an alternative process, propylene oxide is converted to allyl alcohol. The allyl alcohol is then hydroformylated to form 4-hydroxybutyraldehyde, which may be hydrogenated to form 1,4-butanediol. Another industrial process requires maleic anhydride as a starting material and proceeds via conversion to the methyl maleate ester and subsequent hydrogenation. Other traditional routes use butadiene, allyl acetate or succinic acid as starting materials.

In recent years, increased efforts have focused on producing chemicals, including 1,4-BDO, from renewable feedstocks, such as sugar-based materials. US 8067214 describes a biosynthetic pathway to produce 1,4-BDO directly from sugar using a non-naturally occurring microbial organism.

A further method for obtaining 1,4-butanediol from non-fossil fuel based sources involves its production by decarbonylation of furfural and proceeds via an intermediate such as furan. Examples of reaction processes for achieving these steps can be found in Hoydonck, H.E., Van Rhijn, W.M., Van Rhijn, W., De Vos, D.E. & Jacobs, P.A. (2012) Furfural and Derivatives, in Ulmann's Encyclopedia or Industrial Chemistry (volume 16, pp 285-313), Wiley-VCH Verlag GmBH & Co. KGaA, Weinheim; Dunlop, A.P. and Peters, F.N., in The Furans, Reinhold Publ. Co, 1953; K.J. Zeitsch, in "The Chemistry and Technology of Furfural and its Many By-products" Sugar Series 13, Elsevier, 2000; Lange, J-P, van der Heide, E, van Buijtenen, J., and Price, R.; Furfural-A Promising Platform for Lignocellulosic Biofuels; ChemSusChem 2012, 5, 150 - 166; and Watson, J.M., Ind. Eng. Chem. Prod. Res. Develop., 1973, 12(4), 310. Furfural may be obtained from hemicellulose via acid hydrolysis in the liquid phase as well as in the gas phase as described in WO 2002/22593 and WO 2012/041990.

Succinic acid is also available from bio-based resources, as described in Green Chem., 2009, 11, 13, and can be used as a starting material in the production of 1,4-butanediol as well as other related useful chemical building blocks such as γ-butyrolactone (GBL) and tetrahydrofuran (THF).

Many processes provide 1,4-BDO as a product within an aqueous stream, optionally in the presence of other materials such as GBL, THF and n-butanol. Typically, the 1,4-BDO, as well as the GBL, THF and n-butanol if present, are then purified using an energy-intensive distillation process, which may be complicated by the presence of azeotropes.

US 4032583 describes a liquid-liquid extraction process in which 1,4-BDO is recovered in the aqueous raffinate phase and co-products are recovered in a hydrocarbon extract phase. The aqueous raffinate phase is processed further to isolate the 1,4-BDO.

It would, therefore, be advantageous to provide an improved method suitable for the recovery of 1,4-butanediol from such aqueous streams.

### Summary of the Invention

Accordingly, the present invention provides a process for the recovery of 1,4-butanediol from an aqueous stream, said process comprising the steps of providing the aqueous stream, providing a first solvent stream, combining said aqueous stream with said first solvent stream and recovering at least a portion of the 1,4-butanediol from the solvent of the first solvent stream by liquid-liquid extraction, wherein the first solvent is an amine or a combination of amines, or is an alcohol selected from n-butanol and n-pentanol.

### Brief Description of the Drawings

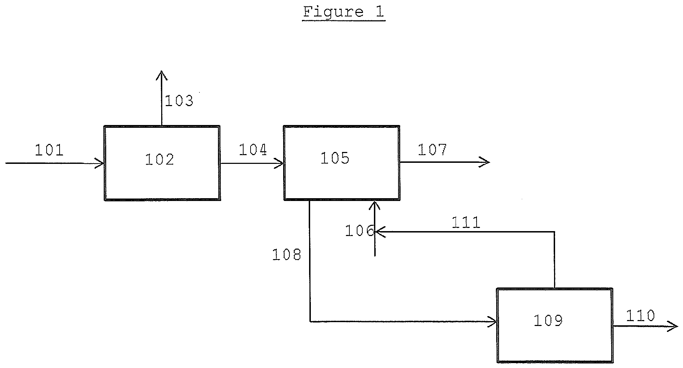

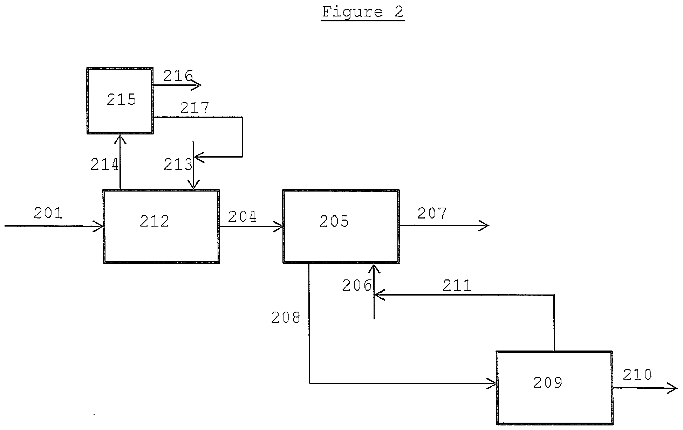

Figures 1 and 2 are schematic diagrams of exemplary, but non-limiting, embodiments of a process for the separation of 1,4-butanediol as described herein.

### Detailed Description of the Invention

The present inventors have surprisingly found that 1,4-butanediol may be individually and independently recovered from an aqueous stream by the use of a solvent in liquid-liquid extraction.

The present invention provides a process for the recovery of 1,4-butanediol from an aqueous stream. The aqueous stream suitably comprises at least 0.1wt%, preferably at least 5wt%, more preferably at least 10wt%, even more preferably at least 20wt%, most preferably at least 25wt% water based on the overall weight of the stream. The aqueous stream suitably comprises at most 99.9wt%, preferably at most 95wt%, more preferably at most 90wt%, most preferably at most 75wt% water based on the overall weight of the stream.

Preferably, the aqueous stream is the reaction product stream from a process for the production of 1,4-butanediol. Particularly preferred reaction product streams include those produced in the processes involving the preparation of 1,4-butanediol by microbial conversion of sugars, the conversion of maleic anhydride to 1,4-butanediol, the conversion of succinic acid to 1,4-butanediol and the conversion of furan to 1,4-butanediol. More preferably, the aqueous stream is the reaction product stream from a process for the production of 1,4-butanediol from bio-based, i.e. non fossil fuel based, feedstocks.

The 1,4-butanediol is typically present in the aqueous stream in an amount of at least 0.1wt%, preferably at least 5wt%, more preferably at least 10wt%, even more preferably at least 25wt%, typically at most 95wt%, preferably at most 90wt%, more preferably at most 80wt%, most preferably at most 75wt%, based on the weight of the overall stream.

As well as the 1,4-butanediol, the aqueous stream may comprise other desirable and non-desirable materials. The nature and quantity of these other materials will depend on the process used to produce the 1,4-butanediol and the conditions used, including catalysts, reaction conditions such as temperature, pressure and starting material and its concentration. Depending on the process used to produce the 1,4-butanediol, the other materials present may include GBL, THF, n-butanol, n-propanol as well as starting materials and other by-products.

The solvent in the first solvent stream suitably comprises a solvent that has a higher affinity for 1,4-butanediol than water and shows a liquid-liquid phase split when mixed with water or saline water at appropriate process temperatures, preferably in the range of from 0 to 250°C. Suitable solvents are nitrogen- or oxygen-containing hydrocarbon-based solvents with these features. Exemplary solvents are those with these features selected from the group of amines, alcohols, furanic compounds such as furfural, esters, ketones, aldehydes and combinations thereof, including compounds containing 2 or more of these functionalities.

In one embodiment of the invention, the solvent in the first solvent stream is an amine. In this embodiment, the solvent in the first solvent stream preferably comprises a primary, a secondary, a tertiary alkyl amine, or a combination thereof. Examples of suitable amines include paraffinic amines, naphthenic amines, aromatic amines, and mixtures thereof. More preferably, the amine is a tertiary amine.

Preferably, the amine contains carbon and nitrogen atoms in a ratio of at most 8:1 (carbon:nitrogen atoms).

Preferably, the amine contains an aliphatic cyclic group either containing the amine nitrogen or attached to the amine nitrogen.

Even more preferably, the amine is a tertiary paraffinic amine. Most preferably, the solvent is selected from the group consisting of N,N-dimethylcyclohexylamine (DMCA), methyl cyclohexyl amine, or N-methyl piperidine, triethylamine, tripropylamine, or a combination thereof.

In another embodiment of the invention, the solvent in the first solvent stream is an alcohol selected from n-butanol and n-pentanol. Also described herein are primary, secondary and tertiary C₄₊ alcohols and mixtures thereof. Other suitable alcohols according to some arrangements may include octanols, including 2-ethylhexanol.

The first solvent stream may be added to or combined with the aqueous stream comprising 1,4-butanediol in any amount sufficient to allow a portion of the 1,4-butanediol to dissolve in the solvent therein. Water may also dissolve in said solvent to the extent that the 1,4-butanediol to water ratio in the extract stream is larger than in the aqueous stream. In certain embodiments, the amount of solvent in the first solvent stream added to or combined with the aqueous stream may be from 10 to 500wt% of the total overall content of that stream.

Preferably, the ratio of solvent in the first solvent stream to 1,4-butanediol may be the minimum amount for exceeding the solubility limit of said solvent in the aqueous stream to less than the amount needed to dissolve the entire aqueous stream. The amount of solvent added to or combined with the aqueous stream may suitably be at least 10wt%, preferably at least 20wt%, more preferably at least 25wt% of the amount of 1,4-butanediol in the aqueous stream. The amount of solvent added to or combined with the aqueous stream may suitably be at most 2000wt%, preferably at most 500wt%, more preferably at most 100wt% of the amount of 1,4-butanediol in the aqueous stream.

The aqueous stream is combined with the solvent stream by any method suitable for the combination of two liquid streams, including but not limited to using a stirred mixer, passing the streams through a static mixer or by agitation. State of the art liquid-liquid contactors (extraction units) are, for example, a series of mixers and settlers, agitated extraction columns, packed extraction columns, SCHEIBEL® Columns, KARR@ Columns, rotating disc contactor (RDC) columns, pulsed, packed (SMVP) and sieve tray columns. In a preferred embodiment of the invention, the two streams are combined in a counter-current extraction unit. In such a unit, the two streams are fed to the unit at points separated by at least 50% of the length, preferably substantially the entire length, of the unit and are brought into contact with each other while passing through the unit in a counter-current fashion.

The 1,4-butanediol is recovered from the aqueous stream by liquid-liquid extraction after the first solvent stream has been added to or combined with said aqueous stream. For example, after the first solvent stream has been added to or combined with the aqueous stream, a portion of the 1,4-butanediol may be extracted into the solvent contained therein. Said solvent, along with the 1,4-butanediol, may then be separated from the rest of the aqueous stream, forming a first 1,4-butanediol and solvent rich stream and a first residual stream.

Preferably, any salt remains dissolved in the aqueous stream so that the separation process happens without precipitation of salts.

In certain embodiments, the liquid-liquid extraction may be enhanced by the inclusion of a synergist. Examples of suitable synergist include demulsifiers. Typical demulsifiers can be phenol-formaldehyde resins, epoxy resins, polyamines, di-epoxides or polyols.

Preferably, the method further comprises recovering the 1,4-butanediol and/or solvent from the first 1,4-butanediol and solvent rich stream. The 1,4-butanediol and/or solvent may be recovered from the first 1,4-butanediol and solvent rich stream through a distillation process. In certain embodiments, the solvent or the 1,4-butanediol may be recovered as the distillate or bottom product. In certain embodiments, the first 1,4-butanediol and solvent rich stream may be distilled to form a first 1,4-butanediol rich stream and a first solvent rich stream. Optionally, the solvent may be recycled.

In one embodiment of the invention, it is preferable that further 1,4-butanediol is then recovered from the first residual stream by the steps of providing a second solvent stream, combining the first residual stream with said solvent stream and recovering at least a portion of the 1,4-butanediol present in the first residual stream by liquid-liquid extraction.

This process may be carried out under the same or different conditions to the recovery of 1,4-butanediol from the aqueous stream. Suitable conditions, equipment, quantities and materials may be selected from the conditions, equipment, quantities and materials detailed above as suitable for the recovery of 1,4-butanediol from the aqueous stream.

In this embodiment of the invention, combining the first residual stream with the second solvent stream may take place in the same or a different extraction unit as the combining of the aqueous stream and the solvent stream.

In this embodiment of the invention, the 1,4-butanediol is recovered from the first residual stream by liquid-liquid extraction after the second solvent stream has been added to or combined with said first residual stream. For example, after the second solvent stream has been added to or combined with the first residual stream, a portion of the 1,4-butanediol may be extracted into the solvent contained therein. Said solvent, along with the 1,4-butanediol, may then be separated from the rest of the first residual stream, forming a second 1,4-butanediol and solvent rich stream and a second residual stream.

Subsequent recovery of further 1,4-butanediol may optionally be carried out in the same manner resulting in third and subsequent 1,4-butanediol and solvent rich streams and third and subsequent residual streams.

In a preferred embodiment of the invention, one or more of the first, second, third and subsequent residual streams may be recycled for use in the process in which the 1,4-butanediol is formed. Additional advantages of such a step include the reduction of the amount of waste produced and requiring disposal and also retaining any remaining 1,4-butanediol and/or desirable by-products in the system for subsequent recovery.

Preferably, in each case the method further comprises recovering the 1,4-butanediol and/or solvent from the second, third and/or any subsequent 1,4-butanediol and solvent rich stream. The 1,4-butanediol and/or solvent may be recovered from the second, third and/or any subsequent 1,4-butanediol and solvent rich stream through a distillation process. In certain embodiments, the solvent or the 1,4-butanediol may be recovered as the distillate or bottom product. In certain embodiments, the second, third and/or any subsequent 1,4-butanediol and solvent rich stream may be distilled to form a second, third and/or subsequent 1,4-butanediol rich stream and a second, third and/or subsequent solvent rich stream. In further embodiments, the second, third and/or any subsequent 1,4-butanediol and solvent rich stream may be combined with other such streams, such as the first 1,4-butanediol and solvent rich streams before distillation is carried out. In each case, optionally, the solvent may be recycled.

The process is preferably carried out at a temperature of at least 5°C, more preferably at least 10°C, even more preferably at least 20°C, even more preferably at least 25°C, most preferably at least 50°C. The temperature is preferably at most 250°C, more preferably at most 200°C, even more preferably at most 150°C. The pressure is preferably in the range of from 0.1 to 10 MPa, more preferably in the range of from 0.1 to 2.5MPa, even more preferably in the range of from 0.1 to 1MPa and must suitably be high enough to avoid vaporisation of the materials.

In a preferred embodiment of the invention, the aqueous stream is derived from a process for the production of 1,4-butanediol. After completion of the process for the production of 1,4-butanediol, said reaction product stream may also contain other desirable products such as GBL and/or THF and/or n-butanol. In a particularly preferred embodiment of the invention, these desirable products are separated from the reaction product stream before the resultant aqueous stream is combined with the first solvent stream. Suitably, known means for separating the THF and/or GBL and/or n-butanol from an aqueous stream may be used for this separation, including, but not limited to, distillation and extraction. In a preferred embodiment of the invention, THF and/or GBL and/or n-butanol, if present, are extracted from the reaction product stream by combining said reaction product stream with a further solvent stream and recovering at least a portion of the THF and/or GBL and/or n-butanol by liquid-liquid extraction.

The further solvent stream suitably comprises a solvent that has a higher affinity for the materials to be extracted than water and shows a liquid-liquid phase split when mixed with water or saline water at appropriate process temperatures, preferably in the range of from 0 to 200°C. Preferably, the solvent in the further solvent stream shows a higher selectivity for THF and/or GBL and/or n-butanol than for 1,4-butanediol. Preferable solvents in the further solvent stream are those with these features selected from the group of alkanes, olefins, aromatics, ethers and esters, such as ethyl acetate. Preferred alkanes include butanes, pentanes, hexanes, heptanes, octanes, nonanes, decanes, higher alkane solvents and mixtures thereof including C5/C6 refinery streams and C6-C9 naptha streams. Particularly preferred alkane solvents include n-pentane and n-decane. Particularly preferred ether solvents include dibutyl ether.

The further solvent stream may be added to or combined with the reaction product stream in any amount sufficient to allow at least a portion of the THF and/or GBL and/or butanol to dissolve in the solvent contained therein. Water may also dissolve in the further solvent stream to the extent that the THF and/or GBL and/or butanol to water ratio in the extracted solvent stream is larger than in the reaction product stream. In certain embodiments, the amount of the solvent in the further solvent stream (herein after the 'further solvent') added to or combined with the reaction product stream may be from 10 to 500wt% of the total overall content of that stream.

Preferably, the ratio of the solvent in the further solvent stream to THF and/or GBL and/or n-butanol, if present, may be the minimum amount for exceeding the solubility limit of the further solvent in the reaction product stream to less than the amount needed to dissolve the entire reaction product stream. The amount of the further solvent added to or combined with the reaction product stream may suitably be at least 10wt%, preferably at least 20wt%, more preferably at least 25wt% of the amount of THF and/or GBL and/or n-butanol, if present, in the reaction product stream. The amount of the further solvent added to or combined with the reaction product stream may suitably be at most 2000wt%, preferably at most 500wt%, more preferably at most 100wt% of the amount of THF and/or GBL in the reaction product stream.

The reaction product stream is combined with the further solvent stream by any method suitable for the combination of two liquid streams, including but not limited to using a stirred mixer, passing the streams through a static mixer or by agitation. State of the art liquid-liquid contactors (extraction units) are, for example, a series of mixers and settlers, agitated extraction columns, packed extraction columns, SCHEIBEL® Columns, KARR® Columns, rotating disc contactor (RDC) columns, pulsed, packed (SMVP) and sieve tray columns. In a preferred embodiment of the invention, the two streams are combined in a counter-current extraction unit. In such a unit, the two streams are fed to the unit at points separated by at least 50% of the length, preferably substantially the entire length, of the unit and are brought into contact with each other while passing through the unit in a counter-current fashion.

The THF and/or GBL and/or n-butanol, if present, are recovered from the reaction product stream by liquid-liquid extraction after the further solvent stream has been added to or combined with said reaction product stream. For example, after the further solvent stream has been added to or combined with the reaction product stream, at least a portion of the THF and/or GBL and/or n-butanol may be extracted into the further solvent. The further solvent, along with the THF and/or GBL and/or n-butanol, if present, may then be separated from the rest of the reaction product stream forming a first THF and/or GBL and/or n-butanol and further solvent rich stream and a first THF and/or GBL and/or n-butanol poor aqueous stream.

Preferably, in this case, the method further comprises recovering the THF and/or GBL and/or n-butanol and/or further solvent from the THF and/or GBL and/or n-butanol and further solvent rich stream. These materials may be recovered individually or in combinations of materials by using a distillation process comprising any number of steps. In certain embodiments, the further solvent or the THF and/or GBL and/or n-butanol or combinations thereof may be recovered as a distillate or a bottom product. Preferably, through one or more distillation processes, the materials are separated into individual streams each rich in one of the compound. Optionally, the second solvent may be recycled.

The THF and/or GBL and/or n-butanol, if present, extraction process may then be repeated one or more times. The resultant first or a subsequent aqueous stream having had these materials removed may then be used as the aqueous stream in the process of the invention for recovery of 1,4-butanediol from an aqueous stream.

In a particularly preferred, but non-limiting, embodiment of the invention illustrated in Figure 1, a reaction product stream 101 is fed to distillation apparatus 102, in order to separate a stream containing THF 103. The resultant aqueous stream 104 is then provided to a counter-current extraction unit 105 in which it is combined with the first solvent stream 106. Liquid-liquid extraction provides a first residual stream 107 and a first 1,4-butanediol and solvent rich stream 108. The 1,4-butanediol and solvent rich stream 108 is then supplied to distillation apparatus 109 to provide a 1,4-butanediol rich stream 110 and a solvent rich stream 111, which is optionally recycled.

In a further preferred, but non-limiting, embodiment illustrated in Figure 2, a reaction product stream 201 is fed to counter-current extraction unit 212, where it is combined with a further solvent stream 213. Liquid-liquid extraction provides a THF and/or GBL and/or n-butanol poor aqueous stream 204 and a THF and/or GBL and/or n-butanol and further solvent rich stream 214. The THF and/or GBL and/or n-butanol and further solvent rich stream 214 is then supplied to distillation unit 215 to provide a THF and/or GBL and/or n-butanol rich stream 216 and a further solvent rich stream 217, which is optionally recycled to stream 213. The distillation unit 215 will contain one or more distillation columns in order to separate the components within the THF and/or GBL and/or n-butanol and further solvent rich stream 214. The aqueous stream 204 is then provided to a counter current extraction unit 205 in which it is combined with a first solvent stream 206. Liquid-liquid extraction provides a first residual stream 207 and a first 1,4-butanediol and solvent rich stream 208. The 1,4-butanediol and solvent rich stream 208 is then, optionally after further extraction of any remaining THF and/or GBL and/or n-butanol, supplied to distillation apparatus 209 to provide a 1,4-butanediol rich stream 210 and a solvent rich stream 211, which is optionally recycled.

### Examples

### Example 1

An aqueous stock solution was prepared containing 7.1 w% tetrahydrofuran (THF) and 16.5 w% 1,4 butanediol (BDO). 5 gram of this solution was contacted with 3 gram of pentane. After separation of the layers, the organic and water phase were analyzed by GC. The organic phase contained 75% of the THF present and virtually no BDO.

### Example 2

Using the stock solution described in example 1. 5 gram of this solution was contacted with 2.3 gram of diethylether. After separation of the layers, the organic and water phase were analyzed by GC. The organic phase contained 80% of the THF present and 1.5% of BDO present.

### Example 3

Using the stock solution described in example 1. 5 gram of this solution was contacted with 2.7 gram of ethyl acetate. After separation of the layers, the organic and water phase were analyzed by GC. The organic phase contained 80% of the THF present and 4.5% of BDO present.

### Example 4

An aqueous stock solution was prepared containing 9.1 w% 1,4 butanediol (BDO). 5 gram of this solution was contacted with 2.4 gram of n-butanol. After separation of the layers, the organic and water phase were analyzed by GC. The organic phase contained 25% of the BDO present.

### Example 5

Using the stock solution described in example 4. 5 gram of this solution was contacted with 2.5 gram of dimethylcyclohexylamine. After separation of the layers, the organic and water phase were analyzed by GC. The organic phase contained 25% of the BDO present.

### Example 6

Using the stock solution described in example 4. 5 gram of this solution was contacted with 2.2 gram of triethylamine. After separation of the layers, the organic and water phase were analyzed by GC. The organic phase contained 14% of the BDO present.

## Claims

1. A process for the recovery of 1,4-butanediol from an aqueous stream, said process comprising the steps of providing the aqueous stream, providing a first solvent stream, combining said aqueous stream with said first solvent stream and recovering at least a portion of the 1,4-butanediol from the solvent of the first solvent stream by liquid-liquid extraction, wherein the first solvent is an amine or a combination of amines, or is an alcohol selected from n-butanol and n-pentanol.

2. A process according to claim 1, wherein the aqueous stream is derived from a reaction product stream from a process for the production of 1,4-butanediol.

3. A process according to claim 2, wherein the reaction product stream also comprises THF, GBL, or n-butanol, or any combination thereof.

4. A process according to claim 3, wherein GBL and/or THF and/or n-butanol are removed from the reaction product stream in order to provide the aqueous stream.

5. A process according to claim 4, wherein THF and/or n-butanol is removed by a process comprising distillation.

6. A process according to claim 4, wherein the GBL and/or THF and/or n-butanol are removed by a process comprising providing the reaction product stream, providing a further solvent stream, combining said reaction product stream with said further solvent stream and recovering at least a portion of the THF and/or GBL and/or n-butanol by liquid-liquid extraction.

7. A process according to any one of claims 1 to 6, wherein at least a portion of the 1,4-butanediol is recovered by liquid-liquid extraction by a process comprising the steps of extracting a portion of the 1,4-butanediol into the solvent and separating a first 1,4-butanediol and solvent rich stream, leaving a first residual stream.

8. A process according to claim 7, wherein the residual stream is recycled back for use in a process for the production of 1,4-butanediol.

9. A process according to claim 7, wherein the 1,4-butanediol and solvent rich stream is then separated into a 1,4-butanediol rich stream and a solvent rich stream by distillation.

10. A process according to any one of claims 1 to 9, wherein the first solvent is a tertiary amine.

11. A process according to any one of claims 1 to 9, wherein the first solvent is selected from the group consisting of dimethyl-cyclohexyl amine, methyl cyclohexyl amine, N-methyl piperidine, triethylamine, tripropylamine, or a combination thereof.

12. A process according to claim 6, wherein the further solvent stream comprises a compound selected from one or more alkanes, olefins, aromatics, ethers and esters.

13. A process according to claim 12, wherein the further solvent stream comprises an alkane selected from butanes, pentanes, hexanes, heptanes, octanes, nonanes, decanes, higher alkane solvents and mixtures thereof.

14. A process according to claim 12, wherein the further solvent stream comprises dibutyl ether.

## Patentansprüche

1. Vorgang zum Wiedergewinnen von 1,4-Butandiol aus einem wässrigen Strom, wobei der Vorgang die folgenden Schritte umfasst: Bereitstellen des wässrigen Stroms, Bereitstellen eines ersten Lösungsmittelstroms, Kombinieren des wässrigen Stroms mit dem ersten Lösungsmittelstrom und Wiedergewinnen wenigstens eines Anteils des 1,4-Butandiols von dem Lösungsmittel des ersten Lösungsmittelstroms durch Flüssig-Flüssig-Extraktion, wobei das erste Lösungsmittel ein Amin oder eine Kombination von Aminen ist, oder ein Alkohol ist, ausgewählt aus n-Butanol und n-Pentanol.

2. Vorgang nach Anspruch 1, wobei der wässrige Strom von einem Umsetzungsproduktstrom von einem Vorgang für die Herstellung von 1,4-Butandiol stammt.

3. Vorgang nach Anspruch 2, wobei der Umsetzungsproduktstrom ebenfalls THF, GBL oder n-Butanol oder eine beliebige Kombination daraus umfasst.

4. Vorgang nach Anspruch 3, wobei GBL und/oder THF und/oder n-Butanol aus dem Umsetzungsproduktstrom entfernt wird, um den wässrigen Strom bereitzustellen.

5. Vorgang nach Anspruch 4, wobei THF und/oder n-Butanol durch einen Vorgang, der Destillation umfasst, entfernt wird.

6. Vorgang nach Anspruch 4, wobei das GBL und/oder das THF und/oder das n-Butanol durch einen Vorgang entfernt wird, der Folgendes umfasst: Bereitstellen des Umsetzungsproduktstroms, Bereitstellen eines weiteren Lösungsmittelstroms, Kombinieren des Umsetzungsproduktstroms mit dem weiteren Lösungsmittelstrom und Wiedergewinnen wenigstens eines Anteils des THF und/oder des GBL und/oder des n-Butanols durch Flüssig-Flüssig-Extraktion.

7. Vorgang nach einem der Ansprüche 1 bis 6, wobei wenigstens ein Anteil des 1,4-Butandiols durch Flüssig-Flüssig-Extraktion durch einen Vorgang wiedergewonnen wird, der die folgenden Schritte umfasst: Abscheiden eines Anteils des 1,4-Butandiols in das Lösungsmittel hinein und Trennen eines ersten 1,4-Butandiols und eines Stroms, der reich an Lösungsmitteln ist, wobei ein erster Reststrom zurückbleibt.

8. Vorgang nach Anspruch 7, wobei der Reststrom zur Verwendung in einem Vorgang zur Herstellung von 1,4-Butandiol zurück recycelt wird.

9. Vorgang nach Anspruch 7, wobei das 1,4-Butandiol und der Strom, der reich an Lösungsmitteln ist, dann durch Destillation in einen Strom, der reich an 1,4-Butandiol ist, und einen Strom, der reich an Lösungsmitteln ist, getrennt wird.

10. Vorgang nach einem der Ansprüche 1 bis 9, wobei das erste Lösungsmittel ein tertiäres Amin ist.

11. Vorgang nach einem der Ansprüche 1 bis 9, wobei das erste Lösungsmittel ausgewählt ist aus der Gruppe bestehend aus Dimethylcyclohexylamin, Methylcyclohexylamin, N-Methylpiperidin, Triethylamin, Tripropylamin oder einer Kombination daraus.

12. Vorgang nach Anspruch 6, wobei der weitere Lösungsmittelstrom eine Verbindung umfasst, ausgewählt aus einem oder mehreren Alkanen, Olefinen, Aromastoffen, Ethern und Estern.

13. Vorgang nach Anspruch 12, wobei der weitere Lösungsmittelstrom ein Alkan umfasst, das ausgewählt ist aus Butanen, Pentanen, Hexanen, Heptanen, Octanen, Nonanen, Decanen, höheren Alkanlösungsmitteln und Mischungen daraus.

14. Vorgang nach Anspruch 12, wobei der weitere Lösungsmittelstrom Dibutylether umfasst.

## Revendications

1. Procédé de récupération de 1,4-butanediol à partir d'un courant aqueux, ledit procédé comprenant les étapes consistant à fournir le courant aqueux, fournir un premier courant de solvant, combiner ledit courant aqueux avec ledit premier courant de solvant et récupérer au moins une partie du 1,4-butanediol à partir du solvant du premier courant de solvant par extraction liquide-liquide, dans lequel le premier solvant est une amine ou une combinaison d'aminés, ou est un alcool choisi parmi le n-butanol et le n-pentanol.

2. Procédé selon la revendication 1, dans lequel le courant aqueux est obtenu à partir d'un courant de produits de réaction provenant d'un procédé de production de 1,4-butanediol.

3. Procédé selon la revendication 2, dans lequel le courant de produits de réaction comprend également du THF, de la GBL ou du n-butanol, ou toute combinaison de ceux-ci.

4. Procédé selon la revendication 3, dans lequel la GBL et/ou le THF et/ou le n-butanol sont éliminés du courant de produits de réaction afin de fournir le courant aqueux.

5. Procédé selon la revendication 4, dans lequel le THF et/ou le n-butanol sont éliminés par un procédé comprenant une distillation.

6. Procédé selon la revendication 4, dans lequel la GBL et/ou le THF et/ou le n-butanol sont éliminés par un procédé consistant à fournir le courant de produit de réaction, fournir un autre courant de solvant, combiner ledit courant de produit de réaction avec ledit autre courant de solvant et récupérer au moins une partie du THF et/ou de la GBL et/ou du n-butanol par extraction liquide-liquide.

7. Procédé selon l'une quelconque des revendications 1 à 6, dans lequel au moins une partie du 1,4-butanediol est récupérée par extraction liquide-liquide par un procédé comprenant les étapes consistant à extraire une partie du 1,4-butanediol dans le solvant et séparer un premier courant riche en 1,4-butanediol et en solvant, ce qui génère un premier courant résiduel.

8. Procédé selon la revendication 7, dans lequel le courant résiduel est recyclé pour être utilisé dans un procédé de production de 1,4-butanediol.

9. Procédé selon la revendication 7, dans lequel le courant riche en 1,4-butanediol et en solvant est ensuite séparé par distillation en un courant riche en 1,4-butanediol et en un courant riche en solvant.

10. Procédé selon l'une quelconque des revendications 1 à 9, dans lequel le premier solvant est une amine tertiaire.

11. Procédé selon l'une quelconque des revendications 1 à 9, dans lequel le premier solvant est choisi parmi le groupe constitué par la diméthylcyclohexylamine, la méthylcyclohexylamine, la N-méthylpipéridine, la triéthylamine, la tripropylamine ou une combinaison de celles-ci.

12. Procédé selon la revendication 6, dans lequel l'autre courant de solvant comprend un composé choisi parmi un(e) ou plusieurs alcanes, oléfines, aromatiques, éthers et esters.

13. Procédé selon la revendication 12, dans lequel l'autre courant de solvant comprend un alcane choisi parmi les butanes, les pentanes, les hexanes, les heptanes, les octanes, les nonanes, les décanes, les solvants à base d'alcanes supérieurs et les mélanges de ceux-ci.

14. Procédé selon la revendication 12, dans lequel l'autre courant de solvant comprend de l'éther dibutylique.
